# EUROPÄISCHE PATENTSCHRIFT

(11) **EP 3 666 210 B1**
(45) Veröffentlichungstag und Bekanntmachung des Hinweises auf die Patenterteilung: **21.02.2024**
(21) Anmeldenummer: 18212612.8
(22) Anmeldetag: 14.12.2018
(51) Int. Cl.: A61B 34/10

(54) **PLANUNGSUNTERSTÜTZUNG FÜR EINEN INTERVENTIONELLEN EINGRIFF SOWIE ENTSPRECHENDE GERÄTE UND COMPUTERPROGRAMME**
PLANNING SUPPORT FOR AN INTERVENTION AS WELL AS CORRESPONDING DEVICES AND COMPUTER PROGRAMS
AIDE À LA PLANIFICATION POUR UNE INTERVENTION DE LA MÉDECINE INTERVENTIONNELLE ET SYSTÈMES ET LOGICIELS CORRESPONDANTS

(43) Veröffentlichungstag der Anmeldung: 17.06.2020
(73) Patentinhaber: Siemens Healthineers AG, 91301 Forchheim (DE)
(72) Erfinder: Breininger, Katharina, 91052 Erlangen (DE); Pfister, Marcus, 91088 Bubenreuth (DE)
(74) Vertreter: Siemens Healthineers Patent Attorneys

(56) Entgegenhaltungen:
- WO-A1-2017/007947
- DE-A1-102016 212 882

## Beschreibung

Die Erfindung betrifft ein Verfahren und ein bildgebendes Gerät zur Planungsunterstützung für einen interventionellen Eingriff, ein entsprechendes Computerprogramm und ein Speichermedium mit einem derartigen Computerprogramm.

Mit den Fortschritten, die heutzutage in der Medizintechnik, insbesondere in der Datenerfassung und Bildgebung sowie der Datenverarbeitung, gemacht werden, ergeben sich neue Möglichkeiten zur Unterstützung beispielsweise eines Arztes oder Chirurgen. Eine solche Unterstützung durch technische und damit objektive und beispielsweise tagesformunabhängige Mittel kann zu einem verbesserten Behandlungserfolg und damit letztlich zum Patientenwohl beitragen, da der jeweilige behandelnde Arzt gegenüber herkömmlichen Methoden beispielsweise mehr und/oder neue Informationen erhält und/oder von bisher manuell durchzuführenden Schritten oder Tätigkeiten entlastet werden kann.

Beispielsweise ist aus der DE 10 2016 212 882 A1 bereits ein Verfahren zur Planungsunterstützung für einen interventionellen Eingriff bekannt. Dabei wird ein dreidimensionaler Bilddatensatz eines Hohlorgans bereitgestellt und segmentiert. Der dreidimensionale Bilddatensatz wird dann mit einem zweidimensionalen Bild einer Führungsvorrichtung überlagert. Anschließend wird eine korrigierte Position eines Abschnitts des Hohlorgans bestimmt. Basierend auf dieser korrigierten Position soll dann eine Verformungsenergie des Hohlorgans in dem Abschnitt für den Fall einer Entfernung der Führungsvorrichtung bestimmt werden.

DE 10 2016 212 882 A1 offenbart ein Verfahren zur Planungsunterstützung bei einem interventionellen Eingriff zum Einsetzen von Gefäßstützen in ein Hohlorgan eines Patienten. Ferner offenbart WO 2017/007947 A1 ein Verfahren zur Herstellung einer patientenspezifischen Prothese, wobei die Verformung eines Hohlorgans, in welches die Prothese eingebracht werden soll, berücksichtigt wird.

Aufgabe der vorliegenden Erfindung ist es, eine technische Hilfestellung zu geben, welche einem Arzt eine komplikationsfreie Durchführung eines interventionellen Eingriffs zum Platzieren eines Hilfselements in einem Hohlorgan ermöglicht. Diese Aufgabe wird erfindungsgemäß durch die Gegenstände der unabhängigen Patentansprüche gelöst. Vorteilhafte Ausgestaltungen und Weiterbildungen der vorliegenden Erfindung sind in den abhängigen Patentansprüchen, in der Beschreibung und in den Figuren angegeben.

Das erfindungsgemäße Verfahren ist ausgebildet zur Planungsunterstützung für einen interventionellen Eingriff zum Platzieren eines Hilfselements in einem Hohlorgan mittels einer Führungsvorrichtung, welche steifer ist als das Hohlorgan. Das Hilfselement kann bevorzugt eine Gefäßstütze (englisch: Stent) für ein Blutgefäß oder - etwa in der Neuroradiologie - ein intrakranialer Stent, aber ebenso beispielsweise eine Klappe oder dergleichen sein. Die Führungsvorrichtung kann beispielsweise ein Draht, eine Sonde, ein Greifer, ein Endoskop und/oder dergleichen mehr sein oder umfassen. Dadurch, dass die Führungsvorrichtung steifer ist, also weniger leicht verbogen oder verformt werden kann, als das Hohlorgan, wird sich bei einem Einbringen oder Einführen oder einer Anwesenheit der Führungseinrichtung in dem Hohlorgan das Hohlorgan verformen, also an eine Form der Führungsvorrichtung anpassen. Als Teil des vorliegenden Verfahrens wird ein 3D-Bilddatensatz erfasst, welcher einen unbeeinflussten Verlauf des Hohlorgans abbildet. Dieser unbeeinflusste Verlauf des Hohlorgans entspricht also dessen Anordnung und Form ohne die Führungsvorrichtung.

Der 3D-Bilddatensatz wird dann segmentiert, wobei ein Modell, also beispielsweise ein virtuelles geometrisches Objekt oder eine virtuelle Repräsentation, des Hohlorgans in dessen unbeeinflusstem Verlauf oder Zustand erzeugt wird. Der 3D-Datensatz kann beispielsweise ein, bevorzugt präoperatives, also vor dem interventionellen Eingriff aufgenommenes, dreidimensionales Computertomographiebild (CT-Bild), ein CT-Angiographiebild, ein Magnetresonanzbild (MR-Bild, MRT-Bild) oder dergleichen sein.

Weiter wird als Teil des vorliegenden Verfahrens dann eine Verformung des Hohlorgans simuliert anhand eines Verlaufs der Führungsvorrichtung in dem Hohlorgan durch eine Verformung des Modells des Hohlorgans. Es wird hier also berücksichtigt, wie die Führungsvorrichtung - tatsächlich oder abgeschätzt oder angenommen - in dem Hohlorgan verläuft oder voraussichtlich verlaufen wird. Dazu kann der Verlauf der Führungsvorrichtung vorgegeben sein oder zunächst bestimmt werden. Bei dem Simulieren beziehungsweise dem Verformen des Modells kann dieses in Überdeckung mit dem vorgegebenen oder bestimmten Verlauf der Führungsvorrichtung gebracht wird. Ist das Hohlorgan beispielsweise ein Blutgefäß, so kann dessen Modell derart verformt werden, dass sich die Führungseinrichtung oder eine Repräsentation oder ein Modell der Führungsvorrichtung gemäß der Simulation, also gemäß dem verformten Modell des Hohlorgans, in diesem befindet, also von dem Hohlorgan umgeben wird.

Je nachdem, auf welche Weise oder mit welchen Mitteln der 3D-Bilddatensatz erzeugt oder erfasst wurde und der Verlauf der Führungsvorrichtung bestimmt wurde, kann eine Registrierung zwischen dem bestimmten Verlauf und dem 3D-Datensatz, insbesondere dem unbeeinflussten Verlauf des Hohlorgans und dem Modell des Hohlorgans, durchgeführt werden. Letztendlich soll eine konsistente relative räumliche Anordnung oder Kombination des 3D-Bilddatensatzes, des Verlaufs der Führungsvorrichtung und des Modells des Hohlorgans erzeugt werden oder gegeben sein. Für eine entsprechende Registrierung können dem Fachmann bekannte Methoden und Verfahren, beispielsweise eine 2D3D- oder 3D3D-Registrierung, verwendet werden.

Für das Simulieren der Verformung kann ein biomechanisches Modell des Hohlorgans vorgegeben sein. Parameter wie beispielsweise eine Flexibilität oder Steifigkeit des Hohlorgans können in Abhängigkeit von einem konkreten Anwendungsfall vorgegeben sein. Entsprechende Werte können beispielsweise aus vorherigen Messungen und/oder Simulationen bekannt sein oder abgeschätzt werden. Ebenso kann beispielsweise eine Adaption oder Verformungskorrektur durchgeführt werden, wozu beispielsweise die Veröffentlichung "Adaption of 3D Models to 2D X-Ray Images during Endovascular Abdominal Aneurysm Repair" von Daniel Toth und Marcus Pfister et al. Hintergrund bietet. Dieses Simulieren, also ein Bestimmen eines - zumindest angenommenen - Verlaufs des Hohlorgans kann bevorzugt durchgeführt werden, bevor das Hilfselement in das Hohlorgan eingebracht wird.

Erfindungsgemäß wird als Teil des vorliegenden Verfahrens automatisch eine ortsaufgelöste Stauchung und/oder Streckung des Hohlorgans in dessen Längserstreckungsrichtung in seinem verformten Verlauf gemäß dem verformten Modell im Vergleich zu dem unbeeinflussten Verlauf des Hohlorgans bestimmt und angegeben. Die ortsaufgelöste Stauchung und/oder Streckung gibt also eine bereichsweise Längenveränderung des Hohlorgans aufgrund der durch die Führungsvorrichtung - und gegebenenfalls anatomische Randbedingungen - verursachten oder bewirkten Verformung an. Diese Längenveränderung des Hohlorgans wird dabei im Allgemeinen nicht homogen sein, beispielsweise aufgrund inhomogener Materialeigenschaften des Hohlorgans in verschiedenen Bereichen, beispielsweise in Form von Kalzifikationen, aufgrund unterschiedlicher Durchmesser in verschiedenen Bereichen des Hohlorgans, aufgrund einer unterschiedlichen Beschaffenheit eines umgebenden Gewebes und/oder dergleichen mehr. Das Angeben der ortsaufgelösten Stauchung und/oder Streckung kann beispielsweise eine entsprechende Datenausgabe, eine - beispielsweis grafische - Markierung oder Kennzeichnung oder dergleichen bedeuten oder umfassen. Es können also beispielsweise Bereiche des Hohlorgans angegeben oder markiert werden, in denen das Hohlorgan bei der Verformung gestaucht wird, und/oder Bereiche des Hohlorgans, in denen dieses bei der Verformung gestreckt wird. Zusätzlich oder alternativ können beispielsweise entsprechende Zahlenwerte und/oder räumliche Angaben, beispielsweise Koordinatenangaben oder dergleichen, angegeben, also beispielsweise ausgegeben oder angezeigt werden. Es ist hier eine Erkenntnis der vorliegenden Erfindung, dass eine Angabe zu der ortsaufgelösten Stauchung und/oder Streckung besonders nützlich ist, da sich besonders stark gestauchte oder gegebenenfalls gestreckte Bereiche, die sich nach einer Entfernung der Führungsvorrichtung wieder strecken beziehungsweise zusammenziehen, also zumindest annähernd in ihre ursprüngliche Gestalt gemäß dem unbeeinflussten Verlauf zurückverformen, als Landungszone für das Hilfselement letztendlich ungeeignet oder suboptimal sind.

Es kann also beispielsweise wenigstens ein Bereich relativ geringer und/oder relativ großer Stauchung und/oder Streckung des Hohlorgans - jeweils bezogen auf ein entlang des verformten Modells bestimmtes, beispielsweise maximales, Ausmaß, also eine Größe oder Stärke, der Streckung und/oder Stauchung des Hohlorgans - als geeignete beziehungsweise ungeeignete Landungszone für das Hilfselement angegeben werden.

Oftmals steht dem jeweiligen behandelnden Arzt eine tatsächlich gemessene Ansicht des Hohlorgans nur in Form des präoperativen 3D-Bilddatensatzes oder zweidimensionaler Angiographien zur Verfügung. Anhand dieses 3D-Datensatzes oder dieser Angiographien mit dem unbeeinflussten Verlauf des Hohlorgans kann der Arzt jedoch typischerweise nicht ohne Weiteres oder nicht zuverlässig erkennen oder ablesen, in welcher Weise sich das Hohlorgan verformen wird und wie sich die relativen räumlichen Lagebeziehungen oder Abstände zwischen verschiedenen Bereichen des Hohlorgans durch das Einführen der Führungsvorrichtung verändern oder verschieben werden. Während des Eingriffes kann beispielsweise auf einem Durchleuchtungsbild oder einer Angiographie lediglich die Führungsvorrichtung, das Hohlorgan selbst hingegen nicht oder nur als Projektion erkennbar sein, sodass sich auch hier kein unmittelbarer Anhaltspunkt für den behandelnden Arzt bezüglich eines Verformungsverhaltens des Hohlorgans und damit bezüglich als Landungszonen geeigneter oder ungeeigneter Bereiche ergibt.

Bereiche, welche einer relativ starken Längenveränderung, also einer Stauchung und/oder Streckung, unterliegen, werden sich nach dem Entfernen der Führungsvorrichtung in der Regel entsprechend relativ stark oder weit bewegen. Würde sich das Hilfselement oder eine Landungszone des Hilfselements in einem solchen Bereich großer Längenveränderung befinden, könnte dies dann nach dem Entfernen der Führungsvorrichtung zu Komplikationen, etwa zu einem Verschieben oder Verrutschen des Hilfselements, führen. Dies wiederum kann potenziell dazu führen, dass das Hilfselement seine eigentliche Aufgabe oder Funktion nicht mehr erfüllt und/oder beispielsweise von dem Hohlorgan abzweigende Gefäße durch das Hilfselement verdeckt oder abgedeckt werden, wodurch eine Versorgung umgebenden Gewebes oder anderer Organe beeinträchtigt werden kann.

Die vorliegende Erfindung ermöglicht vorteilhaft also eine Platzierung des Hilfselements in einem als geeignete Landungszone angegebenen Bereich oder entsprechend eine Vermeidung von Bereichen des Hohlorgans, welche als Landungszone ungeeignet sind. Da dies auf einem objektiv überprüfbaren, vorgegebenen und unabhängigen Simulations- oder Modellierungsvorgang beruht, kann so beispielsweise ein Einfluss einer gegebenenfalls mangelnden Erfahrung des behandelnden Arztes oder einer im Einzelfall gegebenen Beschränkung bei der Bildgebung oder dergleichen auf einen Behandlungserfolg minimiert werden. Besonders vorteilhaft ermöglicht die vorliegende Erfindung es dem jeweiligen behandelnden Arzt, gestützt auf die Angabe der ortsaufgelösten Streckung und/oder Stauchung, oder gegebenenfalls der darauf basierenden Angabe zu der oder den geeigneten beziehungsweise ungeeigneten Landungszonen, das Hilfselement in einer sicheren Position anzuordnen. Dabei kann gegebenenfalls auf eine zusätzliche Kontrastmittelgabe zum Erkennbarmachen des Hohlorgans während des Eingriffs verzichtet werden. Somit kann durch die vorliegende Erfindung gegebenenfalls auch eine Belastung für den jeweiligen Patienten während des Eingriffs reduziert werden.

Eine Landungszone im Sinne der vorliegenden Erfindung ist insbesondere ein Bereich, in welchem ein Ende oder Rand des Hilfselements angeordnet ist oder angeordnet wird. In diesen Randbereichen kann sich eine Längenveränderung des Hohlorgans besonders signifikant auf eine relative Position des Hilfselements in dem Hohlorgan auswirken. Beispielsweise kann bei der Längenveränderung bei dem Zurückverformen ein Druck oder eine Kraft auf den Randbereich oder das Ende des Hilfselements ausgeübt werden und dieses verschieben. Ebenso kann sich das Hilfselement auf mechanische Eigenschaften des Hohlorgans auswirken, sodass also im Bereich der Landungszone ein entsprechender Unterschied in den mechanischen Eigenschaften des Hohlorgans besteht zwischen einem Bereich, in dem sich das Hilfselement befindet und in einem an der Landungszone unmittelbar angrenzenden Bereich, der frei von dem Hilfselement ist. Hier kann es dann entsprechend zu einem inhomogenen Verhalten bei dem Zurückverformen des Hohlorgans kommen, wodurch das Hilfselement ebenfalls verschoben werden kann.

Die ortsaufgelöste Stauchung und/oder Streckung kann absolut oder relativ bestimmt werden. Dementsprechend kann beispielsweise wenigstens ein Schwellenwert für eine maximale Längenveränderung, also Stauchung oder Streckung, des Hohlorgans vorgegeben sein. Wird dieser Schwellenwert lokal, also in einem bestimmten Bereich oder Teilbereich des Hohlorgans, überschritten, so kann dieser Bereich als für eine Landungszone ungeeignet eingestuft oder angegeben werden. Analog kann ein Bereich, in dem der Schwellenwert unterschritten, also nicht überschritten, wird als für eine Landungszone geeignet eingestuft oder angegeben werden. Der Schwellenwert kann dabei als absoluter Wert vorgegeben sein. Ebenso kann der Schwellenwert beispielsweise als relativer Wert, also beispielsweise als Prozentangabe bezogen auf eine entlang des Hohlorgans maximal auftretende Längenveränderung, angegeben sein. Ebenso kann der Schwellenwert beispielsweise einzelfallabhängig dynamisch vorgegeben oder angepasst werden.

Es kann beispielsweise der Fall auftreten, dass es bei einem konkreten Patienten oder Hohlorgan keinen Bereich ohne Längenveränderung gibt. Das Bestimmen oder Angeben eines oder mehrerer Bereiche geringster oder relativ geringer beziehungsweise größter oder relativ großer Längenveränderung kann dann aber dennoch eine bestmögliche Wahl der Landungszone ermöglichen. Beispielsweise können pauschal alle Bereiche, in denen die jeweils lokale Längenveränderung mehr als 50% eines jeweils im Einzelfall entlang des Hohlorgans auftretenden oder bestimmten Maximalwertes beträgt, als Bereiche relativ großer Längenänderung und damit als ungeeignete Landungszone eingestuft oder angegeben werden. Analog können Bereiche, in denen die lokale Längenveränderung beispielsweise weniger als 50% oder 25% der jeweils im Einzelfall in dem jeweiligen Hohlorgan auftretenden oder bestimmten Längenveränderung beträgt, als Bereiche relativ geringer Längenveränderung und damit als für eine Landungszone geeignet eingestuft oder angegeben werden. Besonders vorteilhaft kann dabei eine kontinuierliche oder graduelle Skala vorgegeben sein oder verwendet werden, auf oder gemäß welcher die lokalen oder bereichsweisen Längenveränderung angegeben sind. Dies ermöglicht vorteilhaft eine besonders einfache und intuitive Identifizierung eines als Landungszone geeigneten Bereiches.

In vorteilhafter Ausgestaltung der vorliegenden Erfindung wird wenigstens ein wenigstens zweidimensionales Bild, also ein zweidimensionales oder dreidimensionales Bild oder ein entsprechender Bilddatensatz, welches die Führungsvorrichtung in dem Hohlorgan abbildet, aufgenommen und dem 3D-Bilddatensatz überlagert. Das wenigstens eine wenigstens zweidimensionale Bild, im Folgenden der Einfachheit halber auch als zweidimensionales Bild oder 2D-Bild bezeichnet, kann beispielsweise ein während des Eingriffs aufgenommenes Röntgen-, Angiographie- oder Fluoroskopiebild sein. Ebenso können gegebenenfalls andere Bildgebungsmodalitäten, beispielsweise Ultraschall oder dergleichen, verwendet werden. Wie bereits erwähnt kann für die konsistente Überlagerung zunächst eine Registrierung zwischen dem 3D-Bilddatensatzes und dem zweidimensionalen Bild, also zwischen jeweiligen Koordinatensystemen dieser Bilder oder Daten oder zu deren Erfassen oder Aufnehmen verwendeter Geräte oder Einrichtungen, insbesondere automatisch, durchgeführt werden. Der Verlauf der Führungsvorrichtung wird dann aus dem wenigstens einen wenigstens zweidimensionalen Bild und/oder der Überlagerung bestimmt. Dabei kann eine Segmentierung des 2D-Bildes vorgesehen sein oder durchgeführt werden, um die Führungsvorrichtung von anderen Teilen des 2D-Bildes abzugrenzen. Bei dieser Ausführungsform der vorliegenden Erfindung kann sich die Führungsvorrichtung also bereits in dem Hohlorgan befinden. Der anhand des 2D-Bildes bestimmte Verlauf der Führungsvorrichtung ist dann vorteilhaft der tatsächliche reale Verlauf und somit besonders genau und zuverlässig. Die Führungsvorrichtung kann wie beschrieben beispielsweise ein Draht sein, welcher dann also in dem 2D-Bild auch ohne Kontrastmittel erkennbar und identifizierbar ist. Da die Führungsvorrichtung sich per Definition oder Voraussetzung in dem Hohlorgan befindet, bildet der Verlauf der Führungsvorrichtung eine Randbedingung für die Verformung des Hohlorgans und die entsprechende Simulation oder Modellierung und kann dementsprechend als Input oder Vorgabe in ein entsprechendes Simulationsmodell oder Simulationsprogramm gegeben werden.

In vorteilhafter Weiterbildung der vorliegenden Erfindung wird in dem 3D-Bilddatensatz ein anatomisches Merkmal des Hohlorgans identifiziert, welches auch mit einer für das wenigstens zweidimensionale Bild verwendeten Bildgebungsmodalität sichtbar ist und sich - zumindest voraussichtlich - durch das Einführen der Führungsvorrichtung in das Hohlorgan verschieben oder bewegen wird. Dies kann beispielsweise auf ein weltfestes Koordinatensystem bezogen sein. Das anatomische Merkmal wird dann auch in dem wenigstens zweidimensionalen Bild identifiziert, also erkannt oder detektiert. Weiter wird das verformte Modell dem wenigstens zweidimensionalen Bild überlagert. Zum Ermitteln einer Genauigkeit der Simulation der Verformung wird dann in der Überlagerung aus dem wenigstens zweidimensionalen Bild und dem verformten Modell ein Abstand zwischen dem anatomischen Merkmal aus dem wenigstens zweidimensionalen Bild und demselben anatomischen Merkmal an dem verformten Modell bestimmt. Die Überlagerung aus dem zweidimensionalen Bild und dem verformten Modell kann dabei eine separate Überlagerung sein oder mit der Überlagerung des 3D-Bilddatensatzes kombiniert sein oder werden. Mit anderen Worten können also dem 3D Bilddatensatz sowohl das zweidimensionale Bild als auch das verformte Modell überlagert werden.

Da das zweidimensionale Bild die tatsächliche physische Realität widerspiegelt, kann es als Referenz dienen, gegenüber welcher das verformte Modell bewertet werden kann. Sofern die Simulation, also die Verformung des Modells des Hohlorgans korrekt, also realitätsgetreu, ist, wird in der Überlagerung das anatomische Merkmal an dem verformten Modell sich gerade dort befinden, wo auch das zweidimensionale Bild dieses anatomische Merkmal zeigt. Es kann beispielsweise ein Abstandsschwellenwert vorgegeben sein. Wird dieser Abstandsschwellenwert durch den bestimmten Abstand erreicht oder überschritten, kann eine entsprechende Warnung ausgegeben werden. Durch diese Warnung wird also darauf hingewiesen, dass die Genauigkeit der Simulation oder Modellierung der Verformung zu gering ist, das verformte Modell also nicht zuverlässig oder genau der Realität entspricht. Auf diese Weise kann vorteilhaft verhindert werden, dass der jeweilige behandelnde Arzt von falschen Annahmen oder Voraussetzungen ausgeht, sodass insgesamt eine Wahrscheinlichkeit für einen erfolgreichen Eingriff verbessert werden kann.

Das anatomische Merkmal kann beispielsweise ein besonders dichter Gewebebereich oder ein Übergang zwischen zwei Gewebebereichen oder Gewebearten mit unterschiedlicher Sichtbarkeit, entsprechend einer Kontrastkante oder einem Kontrastsprung in dem zweidimensionalen Bild, eine Gefäßverzweigung oder -abzweigung oder dergleichen sein. Ein solches anatomisches Merkmal kann vorteilhaft nicht nur - auch bei einer Verschiebung - besonders genau und zuverlässig identifiziert oder lokalisiert werden, sondern auch ohne zusätzliche Kontrastmittelgabe oder bereits bei einer reduzierten Kontrastmittelmenge sichtbar sein, wodurch eine Belastung des Patienten oder des Hohlorgans vorteilhaft möglichst gering gehalten werden kann.

In weiterer vorteilhafter Ausgestaltung der vorliegenden Erfindung wird zum Bestimmen des Verlaufs der Führungsvorrichtung in dem Hohlorgan dieser Verlauf basierend auf einer Lage eines vorgegebenen geplanten Einstichpunkts zum Einführen der Führungsvorrichtung in das Hohlorgan relativ zu einer vorgegebenen geplanten Zielregion für das Hilfselement und basierend auf einer vorgegebenen Form und Steifigkeit der Führungsvorrichtung, insbesondere automatisch, abgeschätzt bevor die Führungsvorrichtung in das Hohlorgan eingeführt wird. Mit anderen Worten kann das vorliegend vorgeschlagene Verfahren also vollständig präoperativ durchgeführt werden. Dies ermöglicht es vorteilhaft, die entsprechenden Simulationen, Modellierungen und Abschätzungen besonders detailliert durchzuführen, da entsprechende Berechnungen beispielsweise nicht in Echtzeit vorliegen müssen. So kann vorteilhaft beispielsweise der Eingriff noch besser oder detaillierter und zuverlässiger geplant werden. Beispielsweise kann eine besonders genau auf den jeweiligen Patienten abgestimmte Form und Länge des Hilfselements vor Beginn des eigentlichen Eingriffs ausgewählt werden.

Um den Verlauf der Führungsvorrichtung abzuschätzen können beispielsweise pauschale Angaben oder Vorgaben für eine zwischen dem Einstichpunkt und der Zielregion liegende Gewebeart beispielsweise deren Steifigkeit oder Verformbarkeit verwendet werden. Ebenso können hierfür beispielsweise aus dem 3D-Datensatz oder anderen Untersuchungen oder Messdaten des jeweiligen Patienten vorliegende Daten mit berücksichtigt werden. Insbesondere wenn die Steifigkeit der Führungsvorrichtung - sehr viel - größer ist als die Steifigkeit des Hohlorgans und/oder des umgebenden Gewebes, kann gegebenenfalls ein Verbiegen der Führungsvorrichtung durch Kontakt mit dem Hohlorgan oder durch einen Einfluss oder Druck des umgebenden Gewebes vernachlässigt werden. Im einfachsten Fall kann es beispielsweise möglich sein, den Verlauf der Führungsvorrichtung in dem Hohlorgan abzuschätzen, indem die Führungsvorrichtung oder ein virtuelles Modell der Führungsvorrichtung dem 3D-Datensatz überlagert und gemäß dem Einstichpunkt und der Zielregion ausgerichtet wird. Vorteilhaft kann ein geplanter Pfad, entlang welchem die Führungsvorrichtung von dem Einstichpunkt zu der Zielregion geführt werden soll, um beispielsweise eine dabei auftretende oder geplante Rotation der Führungsvorrichtung bei dem Abschätzen des Verlaufs der Führungsvorrichtung zu berücksichtigen.

In weiterer vorteilhafter Ausgestaltung der vorliegenden Erfindung wird zum Erzeugen des Modells des Hohlorgans dessen Oberfläche durch virtuelle Gitterelemente nachgebildet. Derartige Gitterelemente (Meshes, Mesh-Elemente) können beispielsweise Dreiecke, Vertices, verbindende Kanten und/oder Punkte einer Punktewolke und/oder dergleichen sein oder umfassen. Die ortsaufgelöste Stauchung und/oder Streckung wird dann durch Bestimmung und Vergleich einer Vielzahl von Abständen einander benachbarter der Gitterelemente vor und nach der Verformung bestimmt. Mit anderen Worten wird also für einander entsprechende Paare von Gitterelementen in dem unverformten und dem verformten Modell deren jeweiliger Abstand zueinander bestimmt. Dabei liegt eine umso stärkere Stauchung oder Streckung vor, je kleiner beziehungsweise je größer der Abstand zweier Gitterelemente in dem verformten Modell im Vergleich zu dem Abstand der entsprechenden Gitterelemente in dem unverformten Modell ist. Mit anderen Worten wird also eine bei der modellierten oder simulierten Verformung des Modells des Hohlorgans auftretende Distanzveränderung zwischen gegebenen Punkten oder Gitterelementen des Modells ausgewertet. Die Verformung kann dabei eine prinzipielle Topologie des Modells unverändert lassen, wie dies auch bei dem physisch realen Hohlorgan der Fall ist. Dieses Vorgehen ermöglicht eine besonders einfach und mit relativ geringem Rechenaufwand durchführbare Bestimmung der Stauchung und/oder Streckung, wobei die Ortsauflösung im Wesentlichen nur durch die Auflösung oder einen Detailgrad des Modells begrenzt ist. Die Verwendung benachbarter Gitterelemente gibt dabei jeweils eine lokale Verformung an. Ebenso können jedoch auch nicht unmittelbar benachbarte Gitterelemente verwendet, also deren Abstandsveränderung bestimmt werden. Auf diese Weise kann eine Auflösung der Längenveränderung und somit ein benötigter Rechenaufwand eingestellt oder angepasst werden.

In weiterer vorteilhafter Ausgestaltung der vorliegenden Erfindung wird die Stauchung und/oder Streckung mittels einer FFM-Simulation (FEM: Finite-Elemente-Methode) an dem Modell des Hohlorgans bestimmt. Dies kann bevorzugt vor dem Eingriff durchgeführt werden, wobei dann der genannte abgeschätzte Verlauf der Führungsvorrichtung und das unverformte Modell, welches also den unbeeinflussten Verlauf des Hohlorgans angibt, als Input, also als Ausgangssituation und als Randbedingung, für die FEM-Simulation verwendet oder vorgegeben werden können. Die FEM-Simulation kann zusätzlich oder alternativ zu der anderen vorliegend beschriebenen Methode verwendet werden. Werden beide Methoden angewendet, so kann dies beispielsweise der Verifizierung oder zum Plausibilisieren jeweilige Ergebnisse dienen. Die Verwendung einer FEM-Simulation kann vorteilhaft besonders genaue Ergebnisse liefern. Die Kombinationen mit dem abgeschätzten Verlauf der Führungsvorrichtung kann dabei besonders vorteilhaft sein, da dann typischerweise keinerlei Einschränkungen hinsichtlich der Berechnungsgeschwindigkeit gegeben ist, sodass ein besonders genaues und zuverlässiges Ergebnis ermittelt werden kann. FEM-Simulationen sind als mathematisches Werkzeug aus anderen Anwendungsbereichen hinreichend bekannt. Weitere Hintergrundinformationen hierzu können beispielsweise der Veröffentlichung "Finite element analysis of abdominal aortic aneurysms: Geometrical and structural reconstruction with application of an anisotropic material model" von David Roy, Gerhard A. Holzapfel et al. in IMA Journal of Applied Mathematics (2014) 79, 1011-1026, doi:10:1093/imamat/hxu037 entnommen werden.

In weiterer vorteilhafter Ausgestaltung der vorliegenden Erfindung wird das Simulieren der Verformung des Hohlorgans unter der Randbedingung eines minimalen Energieaufwandes - beispielsweise für das genannte In-Überdeckung-bringen des unverformten Modells mit dem Verlauf der Führungsvorrichtung - durchgeführt. Grundsätzlich kann es ausgehend von dem unbeeinflussten Verlauf mehrere Möglichkeiten geben, wie oder auf welche Weise sich das Hohlorgan in Reaktion auf die eingebrachte Führungsvorrichtung verformen, also an den Verlauf der Führungsvorrichtung anpassen kann. Gemäß einer Erkenntnis der vorliegenden Erfindung kann durch die Randbedingung oder Vorgabe, dass die Verformung unter minimalem Energieaufwand stattfinden soll und/oder das Hohlorgan in seiner verformten Gestalt eine minimale, mit einer zerstörungsfreien Verformung konsistente Energie oder mechanische Spannung aufweisen soll, ein realistisches und physiologisch plausibles Ergebnis erreicht werden. Es können beispielsweise mehrere Verformungsarten oder Verformungspfade simuliert oder modelliert und hinsichtlich eines jeweils dafür benötigten Energieaufwandes oder einer resultierenden Energie eines jeweiligen Endzustandes verglichen werden, wobei dann diejenige Verformungsart und/oder derjenige Endzustand ausgewählt werden kann, welcher die geringste Energie oder den geringsten Energieaufwand aufweist.

Ebenso kann die Verformung beispielsweise schrittweise durchgeführt werden, wobei ausgehend von jedem Zwischenschritt oder Zwischenzustand mehrere mögliche nächste Verformungsschritte sichtlich ihres benötigten Energieaufwandes miteinander verglichen werden können, wobei dann jeweils derjenige der mehreren möglichen nächsten Zwischenschritte ausgewählt wird, welcher den geringsten Energieaufwand aufweist. Dabei können beispielsweise Vorgaben hinsichtlich Materialeigenschaften oder Materialparameter des Hohlorgans und/oder des umgebenden Gewebes als Randbedingungen oder Vorgaben für das Simulieren vorgegeben sein und verwendet werden.

In weiterer vorteilhafter Ausgestaltung der vorliegenden Erfindung wird vor dem Simulieren der Verformung wenigstens ein Fixpunkt, insbesondere der Einstichpunkt zum Einführen der Führungsvorrichtung in das Hohlorgan und/oder eine Bifurkation des Hohlorgans, vorgegeben, der bei der Simulation der Verformung, beispielsweise bezogen auf ein weltfestes Koordinatensystem, lagefest bleibt. Eine Vorgabe solcher Fixpunkte ist dabei besonders vorteilhaft, da hierdurch die Simulation vereinfacht, also ein benötigter Rechenaufwand signifikant reduziert werden kann. Gleichzeitig wird eine Genauigkeit der Simulation nicht signifikant beeinträchtigt, da erfahrungsgemäß davon ausgegangen werden kann, dass sich entsprechende Fixpunkte tatsächlich nicht oder nur in vernachlässigbaren Umfang bewegen.

In weiterer vorteilhafter Ausgestaltung der vorliegenden Erfindung wird, insbesondere anhand des 3D-Bilddatensatzes, eine Inhomogenität, welche eine Flexibilität des Hohlorgans bereichsweise oder lokal beeinflusst detektiert. Für einen der detektierten Inhomogenität entsprechenden Bereich des Modells des Hohlorgans wird dann ein gegenüber einem Rest, also übrigen Bereichen, des Modells erhöhter Steifigkeitswert für das Simulieren der Verformung vorgegeben. Eine derartige Inhomogenität kann beispielsweise eine Kalzifikation sein. Durch das Berücksichtigen solcher Inhomogenitäten kann die Verformung des Hohlorgans besonders realitätsgetreu simuliert werden. Dies wiederum kann vorteilhaft dazu beitragen, dass das Hilfselement letztendlich tatsächlich in einer geeigneten Position endet, in welchem es seine vorgesehene Funktion oder Aufgabe erfüllen kann. Dabei können bevorzugt auch Inhomogenitäten berücksichtigt werden, welche mittels anderer Verfahren oder Bildgebungsmodalitäten oder Messungen detektiert wurden, wodurch die Genauigkeit oder Realitätstreue der Simulation weiter verbessert werden kann.

In weiterer vorteilhafter Ausgestaltung der vorliegenden Erfindung werden zum Angeben der ortsaufgelösten Stauchung und/oder Streckung des Hohlorgans wenigstens ein Bereich relativ geringer und/oder relativ großer Stauchung und/oder Streckung an dem verformten Modell und ein entsprechender Bereich an dem unverformten Modell gemäß einer vorgegebenen Farbskala farbcodiert. Mit anderen Worten werden also in beiden Modellen Bereiche relativ großer Längenveränderungen farblich anders gekennzeichnet als Bereiche relativ kleiner Längenveränderung. Dabei kann eine kontinuierliche Farbskala verwendet werden, vergleichbar einer Heat Map. Dies ermöglicht vorteilhaft eine besonders schnelle und intuitive Erfassung und Bestimmung geeigneter Landungszone. Ebenso können jeweilige Größen der geeigneten oder ungeeigneten Bereiche besonders einfach und intuitiv erfassbar gemacht werden, beispielsweise im Vergleich zu einer tabellarischen Angabe von Zahlenwerten. Beispielsweise können Bereiche relativ großer Längenveränderung in Rottönen, Bereiche mittlerer Längenveränderung in Gelbtönen und Bereiche relativ kleiner Längenveränderung in Grüntönen eingefärbt oder markiert werden.

In weiterer vorteilhafter Ausgestaltung der vorliegenden Erfindung wird zusätzlich eine Flexibilität des Hilfselements und/oder ein an dem Hohlorgan lagefester Verankerungspunkt des Hilfselements an dem Hohlorgan vorgegeben. Es wird dann eine eigenständige Rückverformung des Hohlorgans mit eingesetztem Hilfselement und ohne die Führungsvorrichtung in Abhängigkeit von der bestimmten ortsaufgelösten Streckung und/oder Stauchung sowie der vorgegebenen Flexibilität und/oder dem vorgegebenen Verankerungspunkt simuliert. Anhand dieser Rückverformungssimulation wird dann eine voraussichtliche Verschiebung des Hilfselements in dem Hohlorgan zwischen seiner Position vor und nach der Rückverformung, also vor und nach Entnahme der Führungsvorrichtung aus dem Hohlorgan, bestimmt. Wenn dabei ermittelt wird, dass die voraussichtliche Verschiebung einen vorgegebenen Schwellenwert überschreitet und/oder gemäß der Rückverformungssimulation das Hilfselement oder ein Ende des Hilfselements voraussichtlich in einem als Landungszone ungeeigneten Bereich enden, also zu liegen kommen, wird, kann automatisch eine entsprechende Warnung ausgegeben werden. Hierdurch kann vorteilhaft verhindert werden, dass das Hilfselement in einer ungeeigneten oder unvorhergesehenen Lage oder Position in dem Hohlorgan zu liegen kommt nachdem der Eingriff beendet ist. Ebenso kann vorteilhaft der oder ein weiterer Schwellenwert war also eine maximale zulässige Verschiebung, als zusätzliche Randbedingung für das Angeben oder Bestimmen des oder der als Landungszone geeigneten oder ungeeigneten Bereiches beziehungsweise Bereiche vorgegeben und verwendet werden.

Es kann sich beispielsweise der Fall ergeben, dass aufgrund der eigenständigen Rückverformung des Hohlorgans das Hilfselement bei eingeführter Führungsvorrichtung in einem als Landungszone an sich ungeeigneten Bereich positioniert werden muss, sodass es nach dem Entnehmen der Führungsvorrichtung und der dabei auftretenden Rückverformung des Hohlorgans in einem als Landungszone geeigneten Bereich endet. Ein solches Vorgehen wäre ohne die vorliegende Erfindung kontraintuitiv und nicht zuverlässig anwendbar.

Die Berücksichtigung der Flexibilität und/oder des Verankerungspunktes kann dabei eine Genauigkeit der Rückverformungssimulation verbessern, da sich das Hohlorgan mit dem eingesetzten Hilfselement gegebenenfalls anders verhalten wird als ohne das Hilfselement, also beispielsweise während der Verformung bei dem Einführen der Führungsvorrichtung. Besonders vorteilhaft kann ein Ergebnis der Rückverformungssimulation, also eine simulierte Endposition des Hohlorgans und des Hilfselements, ebenfalls dem 3D-Bilddatensatz überlagert werden, insbesondere vor und/oder während des Eingriffes. Dies kann dem jeweiligen behandelnden Arzt vorteilhaft eine dynamische Optimierung oder Anpassung der Position oder Anordnung des Hilfselements ermöglichen, insbesondere wenn die Rückverformungssimulation nach einer automatisch erfassten oder detektierten Verschiebung oder Lageveränderung des Hilfselements durch den behandelnden Arzt automatisch erneut durchgeführt oder aktualisiert wird.

In weiterer vorteilhafter Ausgestaltung der vorliegenden Erfindung wird ein durch das Hilfselement zu behandelnder, beispielsweise abzustützender oder abzudichtender, Teilbereich des Hohlorgans vorgegeben. Dieser Teilbereich kann also der bereits genannten Zielregion entsprechen, in der das Hilfselement letztendlich positioniert werden soll. In Abhängigkeit von der ortsaufgelösten Stauchung und/oder Streckung wird dann automatisch eine, entsprechend angepasste oder optimierte, Länge des Hilfselements vorgeschlagen. Dabei können eine oder mehrere Randbedingungen vorgegeben sein, die erfüllt werden müssen. Beispielsweise kann als Randbedingung vorgegeben sein, dass der Teilbereich vollständig durch das Hilfselement abgedeckt oder überdeckt sein muss und/oder das Hilfselement in dem Bereich relativ geringer Stauchung und/oder Streckung enden muss und/oder wenigstens ein anatomisches Merkmal, beispielsweise eine Gefäßabzweigung, nicht überdeckt werden darf. Die Länge des Hilfselements, also dessen Größe oder Ausdehnung insbesondere in Längsrichtung des Hohlorgans, kann dann automatisch so lange variiert werden, bis alle diese vorgegebenen Randbedingungen erfüllt sind.

Ebenso können die Randbedingungen mit einer jeweiligen Priorität oder einem Gewicht vorgegeben sein. Durch automatische Variation der Länge und Überprüfung eines Ergebnisses hinsichtlich der Randbedingungen kann dann automatisch eine optimale Länge des Hilfselements bestimmt werden. Auf diese Weise kann unabhängig von einer Erfahrung des behandelnden Arztes ein optimales Behandlungsergebnis sichergestellt werden.

Es sei an dieser Stelle darauf hingewiesen, dass etwaige zur Verdeutlichung hier im Zusammenhang mit dem erfindungsgemäßen Verfahren genannte oder angedeutete chirurgische Schritte explizit nicht Teil des beanspruchten erfindungsgemäßen Verfahrens sind. Das beschriebene Verfahren wird also lediglich insoweit beansprucht, als es keinen chirurgischen Schritt umfasst. Dies steht einer Ausführbarkeit oder Anwendbarkeit des erfindungsgemäßen Verfahrens jedoch nicht entgegen, da wie beschrieben das Verfahren beispielsweise vollständig präoperativ durchgeführt werden kann. Auch wenn das Verfahren vorteilhaft zumindest teilweise während des genannten interventionellen Eingriffs durchgeführt werden kann, bezieht es sich lediglich auf eine entsprechende Steuerung des bildgebenden Geräts oder einer Datenverarbeitungseinrichtung und nicht auf die bei dem Eingriff möglicherweise durchgeführten chirurgischen Schritte.

Ein weiterer Aspekt der vorliegenden Erfindung ist ein bildgebendes Gerät zur Planungsunterstützung für einen interventionellen Eingriff zum Platzieren eines Hilfselements in einem Hohlorgan mittels einer Führungsvorrichtung, welche steifer ist als das Hohlorgan. Das bildgebende Gerät weist dabei eine Erfassungseinrichtung zum Erfassen eines 3D-Bilddatensatzes auf, welcher einen unbeeinflussten Verlauf des Hohlorgans abbildet.

Ein solches Erfassen des 3D-Bilddatensatzes im Sinne der vorliegenden Erfindung kann ein Aufnehmen oder Messen des 3D-Bilddatensatzes, also entsprechender Rohdaten oder Messwerte, bedeuten oder umfassen. Dementsprechend kann die Erfassungseinrichtung beispielsweise eine Bildgebungsmodalität, also beispielsweise eine Strahlenquelle und einen entsprechenden Detektor oder eine Magnet- und Spulenanordnung oder dergleichen, umfassen. Ebenso kann das Erfassen aber ein Abrufen des 3D-Bilddatensatzes aus einem Datenspeicher sein oder bedeuten oder umfassen. De Erfassungseinrichtung kann dann beispielsweise eine Datenverarbeitungseinrichtung oder Teil einer Datenverarbeitungseinrichtung sein, welche den 3D-Bilddatensatzes über eine entsprechende Daten- oder Kommunikationsschnittstelle erfasst, also abruft. Entsprechendes kann auch für ein Erfassen des wenigstens einen wenigstens zweidimensionalen Bildes gelten.

Das erfindungsgemäße bildgebende Gerät weist weiter eine mit der Erfassungseinrichtung gekoppelte Datenverarbeitungseinrichtung auf. Die Erkennungseinrichtung kann dabei beispielsweise Teil der Datenverarbeitungseinrichtung, beispielsweise eine entsprechende Schaltung und/oder ein entsprechendes Programmodul, sein oder umfassen.

Das erfindungsgemäße bildgebende Gerät ist eingerichtet zum Ausführen oder Durchführen zumindest einer Ausführungsform des erfindungsgemäßen Verfahrens. Das erfindungsgemäße bildgebende Gerät kann also insbesondere das im Zusammenhang mit dem erfindungsgemäßen Verfahren genannte bildgebende Gerät sein. Dementsprechend kann das erfindungsgemäße bildgebende Gerät einzelne, einige oder alle der im Zusammenhang mit dem erfindungsgemäßen Verfahren genannten Eigenschaften, Bauteile und/oder Ausgestaltungen aufweisen.

Die Erfassungseinrichtung kann beispielsweise ebenfalls zum Erfassen des genannten, wenigstens zweidimensionalen Bildes ausgebildet und eingerichtet sein. Die Datenverarbeitungseinrichtung kann entsprechend beispielsweise zum Erzeugen des Modells, zum Bestimmen des Verlaufs, zum Simulieren der Verformung, zum automatischen Bestimmen und Angeben der ortsaufgelösten Stauchung und/oder Streckung ausgebildet und eingerichtet sein. Die Datenverarbeitungseinrichtung kann ebenfalls ausgebildet und eingerichtet sein zum Bestimmen und Angeben wenigstens einen Bereiches relativ geringer und/oder relativ großer Stauchung und/oder Streckung als geeignete beziehungsweise ungeeignete Landungszone für das Hilfselement. Entsprechendes gilt für die im Zusammenhang mit den vorteilhaften Ausgestaltungen und Weiterbildungen des erfindungsgemäßen Verfahrens beschriebenen Merkmale.

Ein weiterer Aspekt der vorliegenden Erfindung ist ein Computerprogramm oder Computerprogrammprodukt, also ein Programmcode, umfassend Befehle, die bei einer Ausführung des Computerprogramms durch ein bildgebendes Gerät, insbesondere das erfindungsgemäße bildgebende Gerät, dieses dazu veranlassen, mindestens eine Ausführungsform des erfindungsgemäßen Verfahrens auszuführen, insbesondere teilautomatisch oder vollautomatisch. Das erfindungsgemäße Computerprogramm kodiert oder repräsentiert also die Verfahrensschritte des erfindungsgemäßen Verfahrens. Die Befehle des Computerprogramms können dementsprechend Steuerbefehle oder Steuerungsanweisungen für das bildgebende Gerät sein oder umfassen.

Ein weiterer Aspekt der vorliegenden Erfindung ist ein computerlesbares Speichermedium, auf dem zumindest eine Ausführungsform des erfindungsgemäßen Computerprogramms gespeichert ist.

Zum Ausführen des erfindungsgemäßen Verfahrens kann das erfindungsgemäße bildgebende Gerät, insbesondere dessen Datenverarbeitungseinrichtung, ein erfindungsgemäßes computerlesbares Speichermedium sowie eine damit verbundene Prozessoreinrichtung, beispielsweise einen Mikroprozessor oder Mikrochip oder Mikrocontroller, aufweisen. Diese Prozessoreinrichtung ist dann zum Ausführen des auf dem computerlesbaren Speichermedium gespeicherten Computerprogramms ausgebildet und eingerichtet.

Ein weiterer Aspekt der vorliegenden Erfindung ist ein Datenträgersignal, welches das erfindungsgemäße Computerprogramm überträgt.

Die bisher und im Folgenden angegebenen Eigenschaften und Weiterbildungen des erfindungsgemäßen Verfahrens und des erfindungsgemäßen bildgebenden Systems sowie die entsprechenden Vorteile sind jeweils sinngemäß wechselseitig zwischen diesen und ebenso auf die anderen Aspekte der Erfindung, also auf das erfindungsgemäße Computerprogramm und das erfindungsgemä-ße computerlesbare Speichermedium, übertragbar und umgekehrt. Es gehören also zu der Erfindung auch solche Weiterbildungen des erfindungsgemäßen bildgebenden Geräts, des erfindungsgemäßen Verfahrens, des erfindungsgemäßen Computerprogramms und des erfindungsgemäßen computerlesbaren Speichermediums, welche Ausgestaltungen aufweisen, die hier zur Vermeidung unnötiger Redundanz nicht explizit in der jeweiligen Kombination oder für jeden dieser Aspekte der vorliegenden Erfindung separat beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:
- FIG 1: eine schematische Darstellung eines Abschnitts eines Hohlorgans mit einem eingesetzten Hilfselement;
- FIG 2: einen beispielhaften schematischen Ablaufplan für ein Verfahren zur Planungsunterstützung für einen interventionellen Eingriff zum Platzieren des Hilfselements in dem Hohlorgan mittels einer steifen Führungsvorrichtung;
- FIG 3: eine schematische Darstellung eines bildgebenden Geräts zur Durchführung des Verfahrens;
- FIG 4: eine schematische Überlagerungsdarstellung aus einem Modell eines Hohlorgans in einem unbeeinflussten Verlauf und einem Bild der Führungsvorrichtung in dem Hohlorgan;
- FIG 5: eine schematische Überlagerungsdarstellung des Bildes aus FIG 4 nach Verformung und Anpassung des Modells an einen Verlauf der Führungsvorrichtung;
- FIG 6: eine Überlagerungsdarstellung aus einem präoperativen 3D-CT-Bilddatensatz und einem intraoperativ aufgenommenen 2D-CT-Bild;
- FIG 7: eine schematische Darstellung eines unbeeinflussten und eines verformten Verlaufs eines Hohlorgans;
- FIG 8: eine schematische Darstellung zur Illustration einer inhomogenen Längenveränderung des Hohlorgans aus FIG 7; und
- FIG 9: die schematische Darstellung aus FIG 7 mit gekennzeichneten Bereichen relativ großer und relativ kleiner Längenveränderung.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den Figuren sind gleiche, funktionsgleiche oder einander entsprechende Elemente der Übersichtlichkeit halber jeweils mit den gleichen Bezugszeichen gekennzeichnet, auch wenn es sich um verschiedene Instanzen oder Beispiels der entsprechenden Elemente handeln kann.

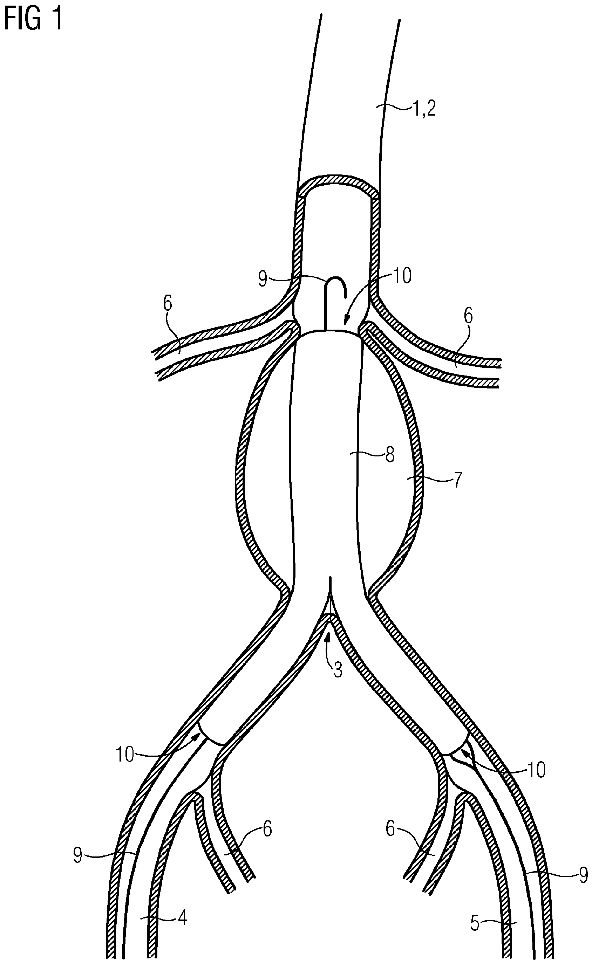

FIG 1 zeigt eine schematische, teilweise geschnittene Ansicht eines Hohlorgans 1. Das Hohlorgan 1 kann hier beispielsweise die gemeinsame Darmbeinarterie 2 (Arteria iliaca communis) und die an einer Bifurkation 3 von dieser abzweigende innere Beckenarterie 4 (Arteria iliaca interna) und äußere Beckenarterie 5 (Arteria iliaca externa) umfassen. Weiter sind hier ausschnittweise einige weitere abzweigende Gefäße 6 dargestellt. Das Hohlorgan 1 weist vorliegend eine krankhafte Gefäßaussackung 7 auf, bei der es sich vorliegend also etwa um ein abdominelles Aortenaneurysma handeln kann. Derartige Gefäßaussackungen 7 können durch Einsetzen einer Gefäßstütze 8 behandelt werden. Die Gefäßstütze 8 kann mittels einer Führungsvorrichtung 9, bei der es sich beispielsweise um einen Draht handeln kann, in dem Hohlorgan 1 im Bereich der Gefäßaussackung 7 positioniert werden. Um eine retrograde Füllung der Gefäßaussackung 7 zu vermeiden, kann die Gefäßstütze 8 über die Bifurkation 3 in die abzweigenden Darmbeinarterien 4, 5 verlängert werden. Endbereiche der Gefäßstütze 8 oder Bereiche oder Abschnitte des Hohlorgans 1, im welchen die Gefäßstütze 8 endet, werden auch als Landungszonen 10 bezeichnet. Offensichtlich ist es zum Behandeln der Gefäßaussackung 7 notwendig, dass die Gefäßstütze 8 im Bereich der Gefäßaussackung 7 positioniert wird. Es hat sich aber herausgestellt, dass auch eine genaue Lage oder Positionierung der Landungszonen 10 einen bedeutenden Einfluss auf einen langfristigen Erfolg der Behandlung der Gefäßaussackung 7 haben kann.

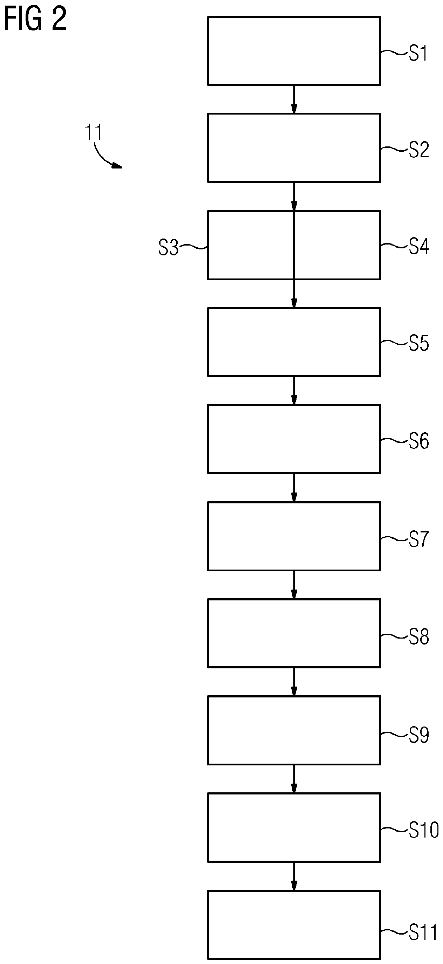

FIG 2 zeigt einen schematischen Ablaufplan 11 für ein Verfahren zur Planungsunterstützung für einen interventionellen Eingriff zum Platzieren eines Hilfselements, beispielsweise der Gefäßstütze 8, in dem Hohlorgan 1 mittels der gegenüber dem Hohlorgan 1 steifen Führungsvorrichtung 9. Dieses Verfahren wird im Folgenden unter Bezugnahme auf die übrigen Figuren näher erläutert.

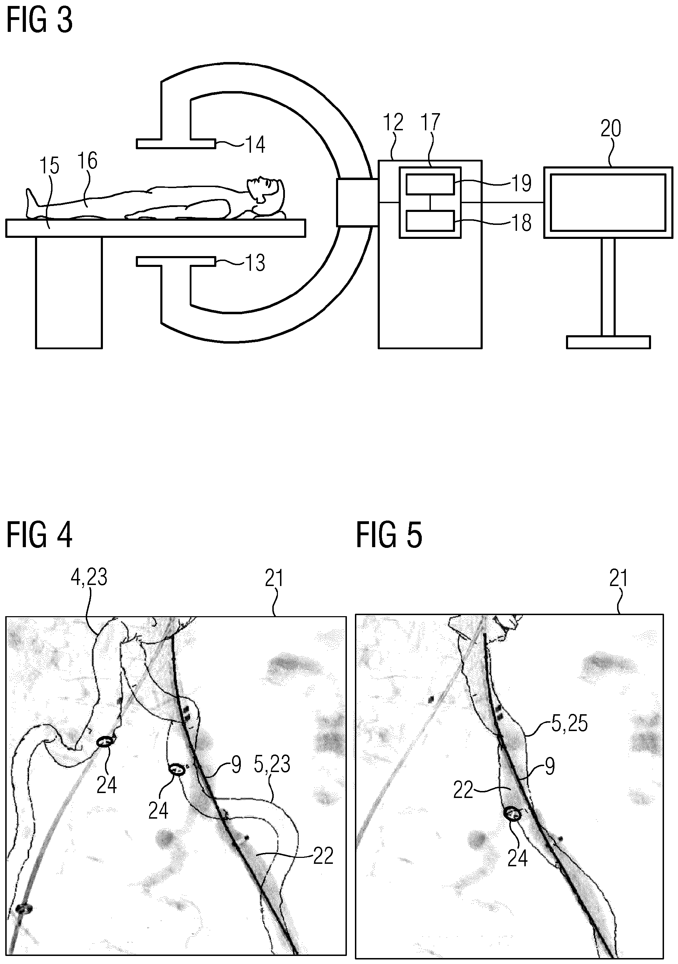

FIG 3 zeigt eine schematische Darstellung eines beispielhaften bildgebenden Geräts 12 zur Durchführung dieses Verfahrens. Das bildgebende Gerät 12 umfasst vorliegend ein C-Bogen-Röntgengerät mit einer Strahlquelle 13 und einem dieser gegenüberliegend angeordneten Detektor 14. Zwischen der Strahlquelle 13 und dem Detektor 14 ist eine Patientenliege 15 angeordnet, auf der sich vorliegend ein abzubildender, zu untersuchender oder zu behandelnder Patient 16 befindet. Weiter weist das bildgebende Gerät 12 vorliegend eine Datenverarbeitungseinrichtung 17 mit einem computerlesbaren Speichermedium 18 und einer damit verbundenen Prozessoreinrichtung 19 auf. Auf dem Speichermedium 18 kann ein Computerprogramm gespeichert sein, welches mittels der Prozessoreinrichtung 19 ausführbar ist und die Verfahrensschritte des Verfahrens zur Planungsunterstützung kodiert oder repräsentiert. Die Verfahrensschritte dieses Verfahrens können also Programmodule oder Funktionsblöcke des entsprechenden Computerprogramms sein.

Die Datenverarbeitungseinrichtung 17 ist vorliegend eingerichtet zum Erfassen und Verarbeiten von beispielsweise mittels der Strahlquelle 13 und des Detektors 14 aufgenommenen Bilddaten, wobei es sich beispielsweise um einen 3D-CT-Bilddatensatz ebenso wie um beispielsweise während des Eingriffs kontinuierlich aufgenommene Angiographie- oder Fluoroskopie Bilder, also Durchleuchtungsbilder, handeln kann. Weiter ist hier eine mit der Datenverarbeitungseinrichtung 17 verbundene Anzeigeeinrichtung 20 vorgesehen zum Anzeigen der Bilddaten oder entsprechender, mittels der Datenverarbeitungseinrichtung 17 erzeugter Bildverarbeitungsergebnisse.

Für den Eingriff zum Positionieren der Gefäßstütze 8 in der Gefäßaussackung 7 kann das bildgebende Gerät 12 also als Angiographieanlage zur Röntgendurchleuchtung des Patienten 16 beziehungsweise des Hohlorgans 1 verwendet werden. Zunächst wird jedoch prä- oder intraoperativ in einem Verfahrensschritt S1 ein 3D-Bilddatensatz eines Bereichs des Hohlorgans 1 erfasst, vorliegend beispielsweise durch die Bildverarbeitungseinrichtung 17. Der 3D-Bilddatensatzes kann mittels des bildgebenden Geräts 12 selbst aufgenommen oder von einer bereitgestellten Datenquelle abgerufen werden. Der 3D-Bilddatensatzes kann insbesondere unter Angabe von Kontrastmittel aufgenommen werden oder aufgenommen worden sein und vorliegend eine wesentliche Anatomie des Hohlorgans 1 und der Gefäßaussackung 7 zeigen.

In einem Verfahrensschritt S2 wird der 3D-Bilddatensatz automatisch, teilautomatisch oder manuell segmentiert. Dabei wird aus dem 3D-Bilddatensatz ein virtuelles geometrisches Modell des Hohlorgans 1 erzeugt. Vorteil eines solchen Modells ist es, dass es sich rechnerisch, also beispielsweise mittels der Datenverarbeitungseinrichtung 17, eleganter, also leichter und schneller verarbeiten und bearbeiten lässt als ein Volumendatensatz, also als der reine 3D-Bilddatensatz. Das erzeugte Modell kann insbesondere Zentrallinien (englisch: Centrelines) abgebildeter Gefäße, vorliegend also beispielsweise der Darmbeinarterien 2, 4, 5 und der abzweigenden Gefä-ße 6, sowie Gitterelemente oder Oberflächenmeshes umfassen, welche eine Oberfläche der Gefäße beziehungsweise des Hohlorgans 1 abbilden oder nachbilden, also modellieren.

Vorliegend sei angenommen, dass der 3D-Bilddatensatz und dessen Segmentieren präoperativ durchgeführt werden. In einem Verfahrensschritt S3 wird dann mittels der Datenverarbeitungseinrichtung 17 ebenfalls präoperativ ein voraussichtlicher Verlauf der Führungsvorrichtung 9 in dem Hohlorgan 1 abgeschätzt. Beispielsweise parallel dazu kann in einem Verfahrensschritt S4 anhand des erzeugten Modells eine FEM-Simulation einer durch das Einbringen der steifen Führungsvorrichtung 9 in das Hohlorgan 1 bewirkten Verformung des Hohlorgans 1 präoperativ durchgeführt werden, um eine Stauchung und/oder Streckung des Hohlorgans 1 bereichsweise, also ortsaufgelöst, zu bestimmen. Die Verfahrensschritte S3 und S4 können optional sein.

In einem Verfahrensschritt S5 kann während des Eingriffs ein wenigstens zweidimensionales Bild eines Bereichs des Hohlorgans 1 aufgenommen oder erfasst werden, welches die Führungsvorrichtung 9 in dem Hohlorgan 1 zeigt oder abbildet.

In einem Verfahrensschritt S6 kann eine Registrierung zwischen dem 3D-Bilddatensatz und dem zweidimensionalen Bild, also zwischen entsprechenden Koordinatensystemen, durchgeführt werden, sofern nicht sowohl der 3D-Datensatz als auch das zweidimensionale Bild mittels desselben bildgebenden Geräts 12 bei unveränderter Stellung oder Lage des Patienten 16 aufgenommen wurden. Das durch das Segmentieren des 3D-Bilddatensatzes erzeugte Modell des Hohlorgans 1 kann in dem Verfahrensschritt S6 dann dem zweidimensionalen Bild in konsistenter, also räumlich korrekter oder realitätsgetreuer Weise überlagert werden. FIG 4 zeigt eine schematische Darstellung eines entsprechenden 2D-Überlagerungsbildes 21, in dem das aus dem präoperativen 3D-Bilddatensatzes erzeugte unverformte Modell 23 des Hohlorgans 1 einem zweidimensionalen Subtraktionsbild überlagert ist. Das zweidimensionale Subtraktionsbild zeigt hier einen Kontrastmittelschatten 22 von Kontrastmittel, welches in das Hohlorgan 1 eingebracht wurde, sowie die in dem Hohlorgan 1 angeordnete Führungsvorrichtung 9. Es ist hier deutlich zu erkennen, dass zwar die Führungsvorrichtung 9 und der Kontrastmittelschatten 22 in ihren Verläufen oder Anordnungen zueinander konsistent sind, dass unverformte Modell 23 hierzu jedoch nicht konsistent ist. Die Führungsvorrichtung 9 würde gemäß dem unverformten Modell 23 das Hohlorgan 1 mehrfach durchstoßen, was tatsächlich nicht der Fall ist, da die Führungsvorrichtung 9 in dem Hohlorgan 1 geführt wird. Vorliegend sind hier an dem unverformten Modell 23 einige anatomische Merkmale 24, welche anhand des 3D-Bilddatensatzes ermittelt wurden, markiert. Bei diesen anatomischen Merkmalen 24 kann es sich beispielsweise um Abgänge der abzweigenden Gefäße 6 oder dergleichen handeln.

In einem Verfahrensschritt S7 kann eine Verformungskorrektur, also eine Simulation oder Modellierung der Verformung des unverformten Modells 23 durchgeführt werden, wodurch dieses in Überdeckung mit dem Verlauf der Führungsvorrichtung 9 gebracht wird. Es wird hierzu also die in das Hohlorgan 1 eingebrachte steife Führungsvorrichtung 9 manuell oder automatisch erkannt und das unverformte Modell, also die entsprechend überlagerte Segmentierung, entsprechend verformt. Dies geschieht insbesondere vor dem Einbringen der Gefäßstütze 8. FIG 5 zeigt hierzu eine schematische Darstellung des 2D-Überlagerungsbildes 21 aus FIG 4 mit dem Unterschied, dass hier nicht das unverformte Modell 23, sondern ein entsprechendes, daraus erzeugtes verformtes Modell 25 des Hohlorgans 1 überlagert ist. Das verformte Modell 25 oder dessen Verlauf ist nun konsistent mit dem Verlauf oder der Anordnung der steifen Führungsvorrichtung 9 und dem Kontrastmittelschatten 22. Die Führungsvorrichtung 9 verläuft nun also in dem verformten Modell 25. Die Verformung des unverformten Modells 23 zu dem verformten Modell 25 kann als Mesh-Verformung durchgeführt werden, bei welcher ursprünglichen Gitterelementen oder Mesh-Elementen des unverformten Modells 23 neue Positionen zugewiesen werden, wobei jedoch eine prinzipielle Topologie des unverformten Modells 23, also auch der entsprechenden Gitterelemente, welche dessen Oberfläche nachbilden oder abbilden, unverändert bleibt. Das unverformte Modell 23 kann also beispielsweise gestaucht, gestreckt oder gedreht werden, nicht jedoch in mehrere Teile aufgetrennt und auf andere Weise wieder zusammengesetzt werden.

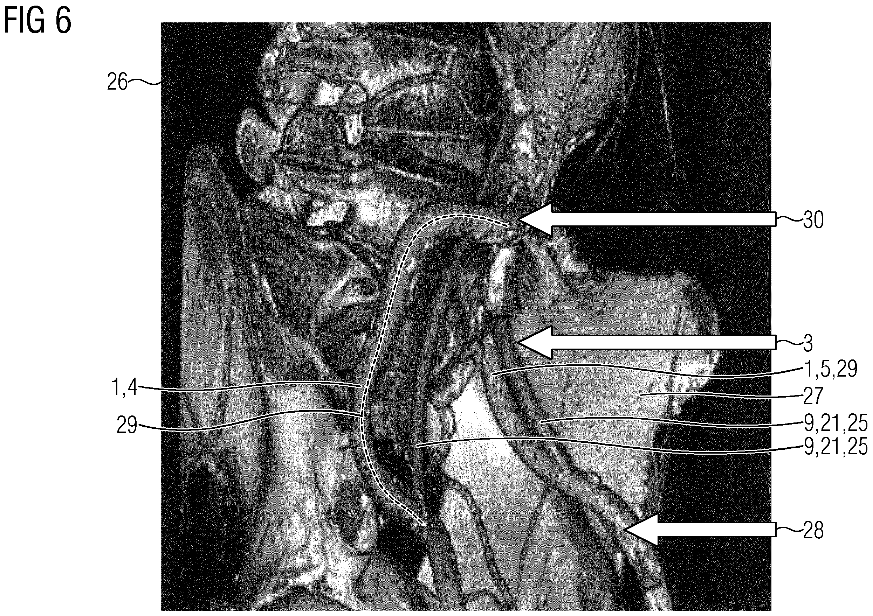

Das in FIG 5 dargestellte 2D-Überlagerungsbild 21 mit dem verformten Modell 25 kann beispielsweise im Verfahrensschritt S7 oder in einem nachfolgenden Verfahrensschritt S8 erzeugt werden. In dem Verfahrensschritt S8 kann ebenso ein schematisch in FIG 6 dargestelltes 3D-Überlagerungsbild 26 aus dem präoperativen 3D-Bilddatensatzes, hier beispielsweise einem 3D-CT-Bild 27 und dem zweidimensionalen Bild oder dem 2D-Überlagerungsbild 21 und beispielsweise den Modellen 23, 25 erzeugt werden. Zur Orientierung sind hier ein Einstichpunkt 28, in welchem die Führungsvorrichtung 9 in das Hohlorgan 1 eingeführt wird, die Bifurkation 3 und eine darüber liegende Aorten-Bifurkation 30 gekennzeichnet. Das 3D-CT-Bild 27 zeigt einen unbeeinflussten Verlauf 29 des Hohlorgans 1, hier beispielsweise der Darmbeinarterien 4, 5. In dem 3D-Überlagerungsbild 26 ist jedoch wie bereits in FIG 4 erkennbar, dass der tatsächliche Verlauf der Führungsvorrichtung 9 in dem Hohlorgan 1 nicht konsistent ist mit dem unbeeinflussten Verlauf 29. Vielmehr verläuft das Hohlorgan 1 tatsächlich entlang der erkennbaren Führungsvorrichtung 9. Es ist hier erkennbar, dass das Einbringen der Führungsvorrichtung 9 in das Hohlorgan 1 zu einer Verkürzung, also einer beispielsweise ziehharmonikaähnlichen Stauchung des Hohlorgans 1 geführt hat. Dies ist zumindest teilweise dadurch bedingt, dass der Einstichpunkt 28 und die Aorten-Bifurkation 30 nicht beliebig beweglich oder bewegbar sind, sondern beispielsweise als lagefeste Fixpunkte fungieren, und die Führungsvorrichtung 9 steifer, also weniger leicht verbiegbar, ist als das Hohlorgan 1.

Beispielsweise anhand des in FIG 5 dargestellten 2D-Überlagerungsbildes 21 und/oder anhand des in FIG 6 dargestellten 3D-Überlagerungsbildes 26 kann in einem Verfahrensschritt S9 eine Genauigkeit der Verformungskorrektur oder der entsprechenden Simulation oder Modellierung der Verformung des Hohlorgans 1 beziehungsweise des unverformten Modells 23 bestimmt oder überprüft werden. Beispielsweise ist in dem 2D-Überlagerungsbild 21 erkennbar, dass bei dem verformten Modell 25 das anatomische Merkmal 24 konsistent zu dem Kontrastmittelschatten 22, also offenbar realitätsgetreu angeordnet ist, während dies bei dem unverformten Modell 23, also in FIG 4, nicht der Fall ist. Ist also ein Abstand zwischen dem anatomischen Merkmal 24 an dem verformten Modell 25 und demselben anatomischen Merkmal in dem zweidimensionalen Bild geringer als ein vorgegebener Abstandsschwellenwert, so kann davon ausgegangen werden, dass die Simulation oder Modellierung der Verformung des unverformten Modells 23 korrekt, also realitätsgetreu, durchgeführt wurde. Damit kann ein behandelnder Arzt mit dem Eingriff auf Basis des verformten Modells 25 fortfahren.

In einem Verfahrensschritt S10 können dann Bereiche relativ großer und/oder relativ kleiner Stauchung und/oder Streckung des Hohlorgans 1 beziehungsweise des verformten Modells 25 bestimmt werden. Beispielsweise können Bereiche höherer oder größerer Stauchung oder Streckung Bereiche oder Abschnitte des verformten Modells 25 sein, in welchen die verformten oder verschobenen, also transformierten, Gitterelemente, beispielsweise Mesh-Vertices, einen geringeren beziehungsweise größeren Abstand zueinander haben als in dem unverformten Modell 23. Danach Freisetzen der Gefäßstütze 8 die Führungsvorrichtung 9 wieder aus dem Hohlorgan 1 entfernt wird, wird dieses weitgehend in seine Ausgangslage zurückgehen, also zumindest im Wesentlichen wieder den unbeeinflussten Verlauf 29 einnehmen. Es kann also davon ausgegangen werden, dass in Bereichen großer Längenveränderung auch entsprechende Rückstellkräfte wirken werden, welche zu einer relativen Verschiebung der Gefäßstütze 8 führen können. Es ist hier vorgesehen, entsprechende Stauchungs- beziehungsweise Streckungsinformationen anhand der Verformung des unverformten Modells 23 zu dem verformten Modell 25 abzuschätzen. Die entsprechenden Bereiche können dann, bevorzugt farbcodiert, markiert oder gekennzeichnet werden.

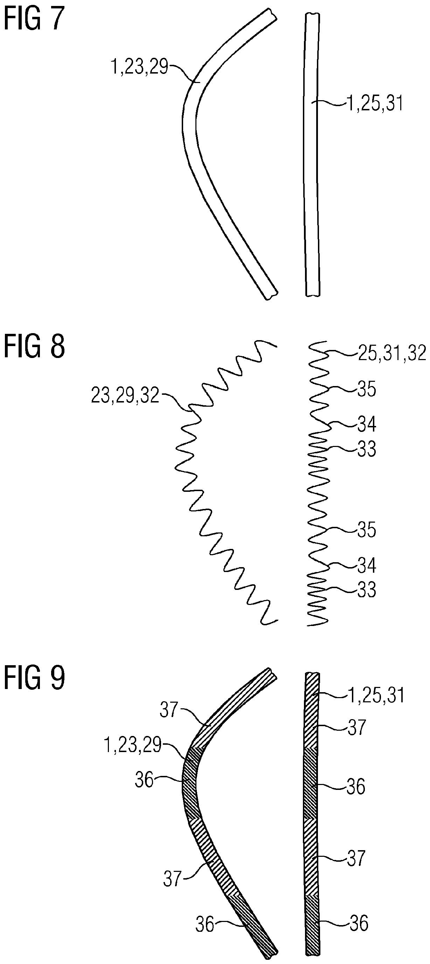

FIG 7 zeigt zur vereinfachten Veranschaulichung eine schematische Darstellung des Hohlorgans 1 in seinem unverformten, also unbeeinflussten Verlauf 29 und seinem verformten Verlauf 31. In dem verformten Verlauf 31 ist das Hohlorgan 1 hier gegenüber dem unbeeinflussten Verlauf 29 verkürzt. Dies kann jedoch in anderen Situationen oder Anwendungsfällen ebenso umgekehrt sein, sodass das Hohlorgan 1 bei der Verformung gegenüber seinem unbeeinflussten Verlauf 29 gestreckt oder verlängert werden könnte. FIG 8 zeigt schematisch, wie das Hohlorgan 1 beispielsweise als Feder 32 modelliert werden kann. In dem unbeeinflussten Verlauf 29 ist die Feder 32 über ihre gesamte Länge hinweg zumindest im Wesentlichen homogen, also gleichmäßig belastet oder gespannt. In dem verformten Verlauf 31 hingegen ist die Feder 32 ungleichmäßig, also inhomogen belastet oder gespannt, also gestaucht oder gestreckt. Deutlich erkennbar sind hier gestauchte Bereiche 33, gestreckte Bereiche 34 und ungestauchte Bereiche 35 dargestellt. Durch Vergleich eines Zustands der Feder 32 in dem unbeeinflussten Verlauf 29 und in dem verformten Verlauf 31 können also entsprechend Bereiche relativ großer beziehungsweise relativ kleiner Stauchung und/oder Streckung bestimmt werden, die sich bei der Verformung von dem unbeeinflussten Verlauf 29 in den verformten Verlauf 31 und entsprechend bei einer Rückverformung aus dem verformten Verlauf 31 in den unbeeinflussten Verlauf 29 ergeben. In FIG 9 sind entsprechend schematisch relativ große Längenveränderungen 36 und relativ geringe Längenveränderungen 37 bereichsweise, also ortsaufgelöst, sowohl in dem verformten Verlauf 31, also an dem verformten Modell 25, als auch in dem unbeeinflussten Verlauf 29, also an dem unverformten Modell 23, markiert oder gekennzeichnet. Dabei können die Längenveränderungen 36, 37 anhand des verformten Verlaufs 31, also anhand des verformten Modells 25 bestimmt und dann durch Zuordnung auf den unbeeinflussten Verlauf 29, also das unverformte Modell 23, übertragen werden. Ebenso kann jedoch zunächst die Rückverformung des Hohlgefäßes 1 beziehungsweise des verformten Modells 25 nach Entfernen der Führungsvorrichtung 9 separat simuliert werden, wobei ein Einfluss der dann eingesetzten Gefäßstütze 8 berücksichtigt werden kann.

In einem Verfahrensschritt S11 können dann als die Landungszonen 10 geeignete Bereiche des Hohlorgans 1 bestimmt, beispielsweise automatisch vorgeschlagen, werden. Ebenso kann hier, bevorzugt automatisch oder teilautomatisch, gegebenenfalls unter Berücksichtigung entsprechender vorgegebener Bedingungen, beispielsweise eine optimale Länge der Gefäßstütze 8 bestimmt und vorgeschlagen werden.

Anschließend kann dann der Eingriff durchgeführt beziehungsweise abgeschlossen, also die Gefäßstütze 8 tatsächlich in das Hohlorgan eingesetzt und gemäß den bestimmten Landungszone 10 optimal in dem Hohlorgan 1 positioniert werden.

Zusammenfassend ist es bei einem derartigen Eingriff also Ziel, die Landungszone 10 einer Gefäßprothese, hier beispielsweise der Gefäßstütze 8, soweit wie möglich in einem gesunden Gefäßwandbereich zu platzieren, dabei aber keine wichtigen Gefäßabgänge, beispielsweise der abzweigenden Gefä-βe 6, zu überdenken. Problematisch kann dabei sein, dass speziell in den stark gekrümmten Iliacalgefäßen relativ starke Verformungen und Längenänderungen, also Stauchungen und/oder Streckungen, durch das Einbringen steifer Instrumente, wie beispielsweise der Führungsvorrichtung 9, auftreten können. Werden diese Instrumente nach Freisetzen der Gefäßprothese wieder entfernt, kann sich das entsprechende Gefäß wieder vollständig oder nahezu vollständig auf eine ursprüngliche Länge ausdehnen oder zusammenziehen. Diese Längenänderungen der Gefäße werden im Allgemeinen nicht homogen sein. Starken oder großen Längenänderungen unterliegende oder unterworfene Gefäßbereiche sind jedoch als die Landungszone 10 für die Gefäßprothese ungeeignet und somit zu vermeiden, um Komplikationen durch eine post-operative Rückverlängerung oder Rückverkürzung zu vermeiden. Da solche Verformungen anhand üblicher 2D-Angiographien nicht oder nur schwer abschätzbar sind, wird hier vorgeschlagen, anhand von prä- und/oder intra-operativen Daten Bereiche abzuschätzen, in denen beispielsweise eine kleinste oder eine größte Längenveränderung stattfindet, um somit Bereiche zu bestimmen, die sich als die Landungszonen 10 eignen beziehungsweise nicht eignen. Dadurch wird eine verbesserte Planung des Eingriffs ermöglicht.

Ein Vorteil dieses Verfahrens besteht darin, über eine präoperative Simulation und/oder eine intra-operative Erkennung eines eingebrachten steifen Drahtes oder dergleichen, hier beispielsweise der Führungsvorrichtung 9, die Bereiche eines Gefäßes, hier also beispielsweise des Hohlorgans 1, abzuschätzen, die zumindest potenziell relativ großen Längenveränderungen unterliegen oder unterliegen werden. Somit ist der Arzt dann in der Lage, für ein Absetzen der Gefäßprothese, beispielsweise eines Iliacalstents, Bereiche mit möglichst geringer Längenveränderung zu wählen, um so ein Migrieren des Stents und/oder andere postoperative Komplikationen zu vermeiden. Dabei kann vorteilhaft gegenüber herkömmlichen Verfahren auch Kontrastmittel eingespart werden.

Das Verfahren kann nicht nur in der hier beschriebenen Weise, sondern prinzipiell für alle Überlagerungen einer Segmentierung angewendet werden.

Insgesamt zeigen die beschriebenen Beispiele wie Landungszonen 10 mit minimaler Längenänderung bestimmt werden können, um eine technische Hilfestellung zu geben, welche einem Arzt eine komplikationsfreie Durchführung eines interventionellen Eingriffs zum Platzieren eines Hilfselements in dem Hohlorgan 1 ermöglicht.

## Patentansprüche

1. Verfahren (11) zur Planungsunterstützung für einen interventionellen Eingriff zum Platzieren eines Hilfselements (8) in einem Hohlorgan (1) mittels einer Führungsvorrichtung (9), welche steifer ist als das Hohlorgan (1), mit den Verfahrensschritten
- Abrufen eines 3D-Bilddatensatzes (27), welcher einen unbeeinflussten Verlauf (29) des Hohlorgans (1) abbildet, aus einem Datenspeicher,
- Erzeugen eines Modells (23) des Hohlorgans (1) in seinem unbeeinflussten Verlauf (29) durch Segmentieren des 3D-Bilddatensatzes (27),
- Simulieren einer Verformung des Hohlorgans (1) anhand eines vorgegebenen oder bestimmten Verlaufs der Führungsvorrichtung (9) in dem Hohlorgan (1) durch eine Verformung des Modells (23, 25), und
- automatisches Bestimmen und Angeben einer ortsaufgelösten Stauchung (33) und/oder Streckung (34) des Hohlorgans (1) in dessen Längserstreckungsrichtung in seinem verformten Verlauf (31) gemäß dem verformten Modell (25) im Vergleich zu dem unbeeinflussten Verlauf (29) des Hohlorgans (1).

2. Verfahren (11) nach Anspruch 1, **dadurch gekennzeichnet, dass** wenigstens ein wenigstens zweidimensionales Bild (21), welches die Führungsvorrichtung (9) in dem Hohlorgan (1) abbildet, aufgenommen und dem 3D-Bilddatensatz (27) überlagert wird und der Verlauf der Führungsvorrichtung (9) aus dem wenigsten einen wenigstens zweidimensionalen Bild (21) und/oder der Überlagerung (26) bestimmt wird.

3. Verfahren (11) nach Anspruch 2, **dadurch gekennzeichnet, dass**
- in dem 3D-Bilddatensatz (27) ein anatomisches Merkmal (24) des Hohlorgans (1) identifiziert wird, welches auch mit einer für das wenigstens zweidimensionale Bild (21) verwendeten Bildgebungsmodalität (12) sichtbar ist und zumindest voraussichtlich durch ein Einführen der Führungsvorrichtung (9) verschiebbar ist,
- das anatomische Merkmal (24) in dem wenigstens zweidimensionalen Bild (21) identifiziert wird,
- das verformte Modell (25) dem wenigstens zweidimensionalen Bild (21) überlagert wird, und
- zum Ermitteln einer Genauigkeit der Simulation der Verformung in der Überlagerung (21) ein Abstand zwischen dem anatomischen Merkmal aus dem wenigstens zweidimensionalen Bild (21) und demselben anatomischen Merkmal (24) an dem verformten Modell (25) bestimmt wird.

4. Verfahren (11) nach einem der vorhergehenden Ansprüche, **dadurch gekennzeichnet, dass** zum Bestimmen des Verlaufs der Führungsvorrichtung (9) in dem Hohlorgan (1) dieser basierend auf einer Lage eines vorgegebenen geplanten Einstichpunkts (28) zum Einführen der Führungsvorrichtung (9) in das Hohlorgan (1) relativ zu einer vorgegebenen geplanten Zielregion (7) für das Hilfselement (8) und basierend auf einer vorgegebenen Form und Steifigkeit der Führungsvorrichtung (9) vollständig präoperativ abgeschätzt wird.

5. Verfahren (11) nach einem der vorhergehenden Ansprüche, **dadurch gekennzeichnet, dass**
- zum Erzeugen des Modells (23) des Hohlorgans (1) dessen Oberfläche durch virtuelle Gitterelemente nachgebildet wird, und
- die ortsaufgelöste Stauchung und/oder Streckung durch Bestimmung und Vergleich einer Vielzahl von Abständen einander benachbarter der Gitterelemente vor und nach der Verformung bestimmt wird.

6. Verfahren (11) nach einem der vorhergehenden Ansprüche, **dadurch gekennzeichnet, dass** die Stauchung und/oder Streckung mittels einer FEM-Simulation an dem Modell (23) des Hohlorgans (1) bestimmt wird.

7. Verfahren (11) nach einem der vorhergehenden Ansprüche, **dadurch gekennzeichnet, dass** das Simulieren der Verformung des Hohlorgans (1) unter der Randbedingung eines minimalen Energieaufwandes durchgeführt wird.

8. Verfahren (11) nach einem der vorhergehenden Ansprüche, **dadurch gekennzeichnet, dass** vor dem Simulieren der Verformung wenigstens ein Fixpunkt, insbesondere ein Einstichpunkt (28) zum Einführen der Führungsvorrichtung (9) in das Hohlorgan (1) und/oder eine Bifurkation (3, 30) des Hohlorgans (1), vorgegeben wird, der bei der Simulation und der Verformung lagefest bleibt.

9. Verfahren (11) nach einem der vorhergehenden Ansprüche, **dadurch gekennzeichnet, dass**, insbesondere anhand des 3D-Bilddatensatzes (27), eine Inhomogenität, welche eine Flexibilität des Hohlorgans (1) bereichsweise beeinflusst, insbesondere eine Kalzifikation, detektiert und für einen der Inhomogenität entsprechenden Bereich des Modells (23) ein gegenüber einem Rest des Modells (23) erhöhter Steifigkeitswert für das Simulieren der Verformung vorgegeben wird.

10. Verfahren (11) nach einem der vorhergehenden Ansprüche, **dadurch gekennzeichnet, dass** zum Angeben der ortsaufgelösten Stauchung und/oder Streckung jeweils bezogen auf ein entlang des verformten Modells (25) bestimmtes Ausmaß der Stauchung und/oder Streckung des Hohlorgans (1) wenigstens ein Bereich relativ geringer (37) und/oder relativ großer (36) Stauchung und/oder Streckung an dem verformten Modell (25) und ein entsprechender Bereich an dem unverformten Modell gemäß einer vorgegebenen Farbskala farbcodiert wird.

11. Verfahren (11) nach einem der vorhergehenden Ansprüche, **dadurch gekennzeichnet, dass**
- zusätzlich eine Flexibilität des Hilfselements (8) und/oder ein an dem Hohlorgan (1) lagefester Verankerungspunkt des Hilfselements (8) an dem Hohlorgan (1) vorgegeben wird,
- eine eigenständige Rückverformung des Hohlorgans (1) mit eingesetztem Hilfselement (8) und ohne die Führungsvorrichtung (9) in Abhängigkeit von der bestimmten ortsaufgelösten Streckung und/oder Stauchung sowie der vorgegebenen Flexibilität und/oder dem vorgegebenen Verankerungspunkt, simuliert wird,
- anhand dieser Rückverformungssimulation eine voraussichtliche Verschiebung des Hilfselements (8) in dem Hohlorgan (1) zwischen seiner Position vor und nach der Rückverformung bestimmt wird.

12. Verfahren (11) nach einem der vorhergehenden Ansprüche, **dadurch gekennzeichnet, dass** ein durch das Hilfselement (8) zu behandelnder Teilbereich (7) des Hohlorgans (1) vorgegeben wird und in Abhängigkeit von der ortsaufgelösten Stauchung und/oder Streckung automatisch eine Länge des Hilfselements (8) vorgeschlagen wird.

13. Bildgebendes Gerät (12) zur Planungsunterstützung für einen interventionellen Eingriff zum Platzieren eines Hilfselements (8) in einem Hohlorgan (1) mittels einer Führungsvorrichtung (9), welche steifer ist als das Hohlorgan (1), wobei das bildgebende Gerät
- eine Erfassungseinrichtung (13, 14, 17) zum Erfassen eines 3D-Bilddatensatzes (27) aufweist, welcher einen unbeeinflussten Verlauf des Hohlorgans (1) abbildet,
- eine mit der Erfassungseinrichtung (13, 14, 17) gekoppelte Datenverarbeitungseinrichtung (17) aufweist, und
- eingerichtet ist zum Ausführen eines Verfahrens (11) nach einem der Ansprüche 1 bis 12.

14. Computerprogramm (11), umfassend Befehle, die bei einer Ausführung des Computerprogramms (11) durch ein bildgebendes Gerät (12) nach Anspruch 13 dieses dazu veranlassen, ein Verfahren (11) nach einem der Ansprüche 1 bis 12 auszuführen.

15. Computerlesbares Speichermedium (18), auf dem ein Computerprogramm (11) nach Anspruch 14 gespeichert ist.

## Claims

1. Method (11) for planning support for an interventional procedure for placing an auxiliary element (8) in a hollow organ (1) by means of a guide facility (9), which is stiffer than the hollow organ (1), with the method steps
- acquisition of a 3D image dataset (27), which maps an uninfluenced course (29) of the hollow organ (1), from a data memory,
- creation of a model (23) of the hollow organ (1) in its uninfluenced course (29) by segmentation of the 3D image dataset (27),
- simulation of a deformation of the hollow organ (1) on the basis of a predetermined or determined course of the guide facility (9) in the hollow organ (1) through a deformation of the model (23, 25), and
- automatic determination and specification of a spatially resolved compression (33) and/or stretching (34) of the hollow organ (1) in its direction of longitudinal extent in its deformed course (31) in accordance with the deformed model (25) compared to the uninfluenced course (29) of the hollow organ (1).

2. Method (11) according to claim 1, **characterised in that** at least one at least two-dimensional image (21), which maps the guide facility (9) in the hollow organ (1), is recorded and is superimposed on the 3D image dataset (27) and the course of the guide facility (9) is determined from the at least one at least two-dimensional image (21) and/or the superimposition (26) .

3. Method (11) according to claim 2, **characterised in that**
- in the 3D image dataset (27) an anatomical feature (24) of the hollow organ (1) is identified, which is also visible with an imaging modality (12) used for the at least two-dimensional image (21) and is at least likely able to be displaced by an introduction of the guide facility (9),
- the anatomical feature (24) is identified in the at least two-dimensional image (21),
- the deformed model (25) is superimposed on the at least two-dimensional image (21), and
- to establish an accuracy of the simulation of the deformation in the superimposition (21), a distance between the anatomical feature is determined from the at least two-dimensional image (21) and the same anatomical feature (24) on the deformed model (25).

4. Method (11) according to one of the preceding claims, **characterised in that**, to determine the course of the guide facility (9) in the hollow organ (1), this is estimated on an entirely preoperative basis based on a location of a predetermined planned insertion point (28) for feeding the guide facility (9) into the hollow organ (1) relative to a predetermined planned target region (7) for the auxiliary element (8) and based on a predetermined shape and stiffness of the guide facility (9).

5. Method (11) according to one of the preceding claims, **characterised in that**
- to create the model (23) of the hollow organ (1), its surface is emulated by virtual mesh elements, and
- the spatially resolved compression and/or stretching is determined by determination and comparison of a plurality of distances between mesh elements adjacent to each other before and after the deformation.

6. Method (11) according to one of the preceding claims, **characterised in that** the compression and/or stretching is determined by means of an FEM simulation on the model (23) of the hollow organ (1).

7. Method (11) according to one of the preceding claims, **characterised in that** the simulation of the deformation of the hollow organ (1) is carried out under the boundary condition of a minimal expenditure of energy.

8. Method (11) according to one of the preceding claims, **characterised in that**, before the simulation of the deformation, at least one fixed point, in particular an insertion point (28) for feeding the guide facility (9) into the hollow organ (1) and/or a bifurcation (3, 30) of the hollow organ (1), is predetermined, which remains in a fixed position during the simulation and the deformation.

9. Method (11) according to one of the preceding claims, **characterised in that**, in particular on the basis of the 3D image dataset (27), an inhomogeneity, which influences a flexibility of the hollow organ (1) area-by-area, in particular a calcification, is detected, and for an area of the model (23) corresponding to the inhomogeneity, an increased stiffness value is predetermined for the simulation of the deformation compared to a remainder of the model (23).

10. Method (11) according to one of the preceding claims, **characterised in that**, to specify the spatially resolved compression and/or stretching related in each case to an extent of the compression and/or stretching of the hollow organ (1) determined along the deformed model (25), at least one area of relatively low (37) and/or relatively great (36) compression and/or stretching on the deformed model (25) and a corresponding area on the undeformed model is colour coded in accordance with a predetermined colour scale.

11. Method (11) according to one of the preceding claims, **characterised in that**
- in addition a flexibility of the auxiliary element (8) and/or an anchoring point of the auxiliary element (8) at a fixed location on the hollow organ (1) is predetermined on the hollow organ (1),
- a discrete reformation of the hollow organ (1) with inserted auxiliary element (8) and without the guide facility (9) is simulated as a function of the spatially resolved stretching and/or compression determined and of the predetermined flexibility and/or the predetermined anchoring point,
- on the basis of this reformation simulation a likely displacement of the auxiliary element (8) in the hollow organ (1) between its position before and after the reformation is determined.

12. Method (11) according to one of the preceding claims, **characterised in that** a part area (7) of the hollow organ (1) to be treated by the auxiliary element (8) is predetermined and, depending on the spatially resolved compression and/or stretching, a length of the auxiliary element (8) is suggested automatically.

13. Imaging device (12) for planning support for an interventional procedure for placing an auxiliary element (8) in a hollow organ (1) by means of a guide facility (9), which is stiffer than the hollow organ (1), wherein the imaging device
- maps an acquisition device (13, 14, 17) for acquisition of a 3D image dataset (27), which maps an uninfluenced course of the hollow organ (1),
- has a data processing device (17) coupled to the acquisition device (13, 14, 17), and
- is configured to carry out a method (11) according to one of claims 1 to 12.

14. Computer program (11), comprising commands that, when the computer program (11) is executed by an imaging device (12) according to claim 13, causes said device to carry out a method (11) according to one of claims 1 to 12.

15. Computer-readable storage medium (18), on which a computer program (11) according to claim 14 is stored.

## Revendications

1. Procédé (11) d'assistance à la planification d'une opération interventionnelle de placement d'un élément (8) auxiliaire dans un organe (1) creux au moyen d'un dispositif (9) de guidage, qui est plus rigide que l'organe (1) creux, comprenant les stades de procédé
- extraction d'une mémoire de données d'un ensemble (27) de données d'image en 3D, qui reproduit un tracé (29) non influencé de l'organe (1) creux,
- production d'un modèle (23) de l'organe (1) creux dans son tracé (29) non influencé par segmentation de l'ensemble (27) de données d'image en 3D,
- simulation d'une déformation de l'organe (1) creux à l'aide d'un tracé donné à l'avance ou déterminé du dispositif (9) de guidage dans l'organe (1) creux par une déformation du modèle (23, 25), et
- détermination automatique et indication d'une compression (33) et/ou d'une extension (34) à résolution spatiale de l'organe (1) creux dans sa direction d'étendue longitudinale dans son tracé (31) déformé suivant le modèle (25) déformé en comparaison du tracé (29) non influencé de l'organe (1) creux.

2. Procédé (11) suivant la revendication 1, **caractérisé en ce que** l'on reproduit, enregistre et superpose, à l'ensemble (27) de données d'image en 3D, au moins une image (21) en au moins deux dimensions, qui reproduit le dispositif (9) de guidage dans l'organe (1) creux, et on détermine le tracé du dispositif (9) de guidage, à partir de la au moins une image (21) en au moins deux dimensions et/ou de le superposition (26).

3. Procédé (11) suivant la revendication 2, **caractérisé en ce que**
- on identifie, dans l'ensemble (27) de données d'image en 3D, une caractéristique (24) anatomique de l'organe (1) creux, qui est visible également avec une modalité (12) d'imagerie utilisée pour l'image (21) en au moins deux dimensions et qui peut être déplacée probablement par une introduction du dispositif (9) de guidage,
- on identifie la caractéristique (24) anatomique dans l'image (21) en au moins deux dimensions,
- on superpose le modèle (25) déformé à l'image (21) en au moins deux dimensions, et
- pour la détermination d'une exactitude de la simulation de la déformation dans la superposition (21), on détermine une distance entre la caractéristique anatomique, dans la au moins une image (21) en deux dimensions, et la même caractéristique (24) anatomique sur le modèle (25) déformé.

4. Procédé (11) suivant l'une des revendications précédentes, **caractérisé en ce que**, pour la détermination du tracé du dispositif (9) de guidage dans l'organe (1) creux, on évalue pré-opérativement complètement celui-ci sur la base d'une position d'un point (28) de piqûre planifiée donnée à l'avance d'introduction du dispositif (9) de guidage dans l'organe (1) creux par rapport à une région (7) cible planifiée donnée à l'avance pour l'élément (8) auxiliaire et sur la base d'une forme et d'une rigidité donnée à l'avance du dispositif (9) de guidage.

5. Procédé (11) suivant l'une des revendications précédentes, **caractérisé en ce que**
- pour la production du modèle (23) de l'organe (1) creux, on reproduit sa surface par des éléments virtuels de réseau, et
- on détermine la compression et/ou l'extension à résolution spatiale par détermination et comparaison d'une pluralité de distances entre des éléments voisins de réseau avant et après la déformation.

6. Procédé (11) suivant l'une des revendications précédentes, **caractérisé en ce que** l'on détermine la compression et/ou l'extension au moyen d'une simulation FEM sur le modèle (23) de l'organe (1) creux.

7. Procédé (11) suivant l'une des revendications précédentes, **caractérisé en ce que** l'on effectue la simulation de la déformation de l'organe (1) creux dans les conditions aux limites d'une dépense minimum d'énergie.

8. Procédé (11) suivant l'une des revendications précédentes, **caractérisé en ce que**, avant la simulation de la déformation, on prescrit au moins un point fixe, en particulier un point (28) de piqûre pour l'introduction du dispositif (9) de guidage dans l'organe (1) creux et/ou une bifurcation (3, 30) de l'organe (1) creux, qui reste fixe en position lors de la simulation et de la déformation.

9. Procédé (11) suivant l'une des revendications précédentes, **caractérisé en ce que**, en particulier à l'aide de l'ensemble (27) de données d'image en 3D, on détecte un défaut d'homogénéité, qui influence, par endroits, une souplesse de l'organe (1) creux, en particulier une calcification, et pour une partie correspondant à un défaut d'homogénéité du modèle (23), on prescrit, pour la simulation de la déformation, une valeur de rigidité plus grande, par rapport à un reste du modèle (23).

10. Procédé (11) suivant l'une des revendications précédentes, **caractérisé en ce que**, pour l'indication de la compression et/ou de l'extension rapportée respectivement à une mesure, déterminée le long du modèle (25) déformé, de la compression et/ou de l'extension de l'organe (1) creux, on code en couleurs, suivant une échelle de couleur donnée à l'avance, au moins une partie de compression et/ou d'extension relativement petite (37) et/ou relativement grande (36) sur le modèle (25) déformé et une partie correspondante sur le modèle non déformé.

11. Procédé (11) suivant l'une des revendications précédentes, **caractérisé en ce que**,
- l'on prescrit en outre une souplesse de l'élément (8) auxiliaire et/ou un point d'ancrage, fixe en position sur l'organe (1) creux, de l'élément (8) auxiliaire sur l'organe (1) creux,
- l'on simule un retour de déformation indépendant de l'organe (1) creux, alors que l'élément (8) auxiliaire est inséré et sans le dispositif (9) de guidage, en fonction de la compression et/ou de l'extension à résolution spatiale déterminée, ainsi que de la souplesse donnée à l'avance et/ou du point d'ancrage donné à l'avance,
- à l'aide de cette simulation de déformation en retour, on détermine un déplacement probable de l'élément (8) auxiliaire dans l'organe (1) creux entre sa position avant et après la déformation en retour.

12. Procédé (11) suivant l'une des revendications précédentes, **caractérisé en ce que** l'on prescrit une zone (7) partielle, à traiter par l'élément (8) auxiliaire, de l'organe (1) creux et on propose automatiquement une longueur de l'élément (8) auxiliaire en fonction de la compression et/ou de l'extension à résolution spatiale.

13. Appareil (12) d'imagerie pour l'assistance à la planification d'une opération interventionnelle de placement d'un élément (8) auxiliaire dans un organe (1) creux au moyen d'un dispositif (9) de guidage, qui est plus rigide que l'organe (1) creux, dans lequel l'appareil d'imagerie
- a un dispositif (13, 14, 17) de saisie pour la saisie d'un ensemble (27) de données d'image en 3D, qui reproduit un tracé non influencé de l'organe (1) creux,
- a un dispositif (17) de traitement de données relié au dispositif (13, 14, 17) de saisie, et
- est agencé pour la réalisation d'un procédé (11) suivant l'une des revendications 1 à 12.

14. Programme (11) d'ordinateur, comprenant des instructions qui, lors d'une réalisation du programme (11) d'ordinateur par un appareil (12) d'imagerie suivant la revendication 13, font que celui-ci effectue un procédé (11) suivant l'une des revendications 1 à 12.

15. Support (18) de mémoire, déchiffrable par ordinateur, sur lequel est mis en mémoire un programme (11) d'ordinateur suivant la revendication 14.
